Europäisches Patentamt

(19) European Patent Office

Office européen des brevets

(11) Publication number: **0 142 987**
**A2**

## (12) EUROPEAN PATENT APPLICATION

(21) Application number: **84307921.1**

(22) Date of filing: **15.11.84**

(51) Int. Cl.⁴: **C 12 N 5/00**

(30) Priority: **17.11.83 GB 8330680**

(43) Date of publication of application:
**29.05.85 Bulletin 85/22**

(84) Designated Contracting States:
**DE FR GB**

(71) Applicant: NATIONAL RESEARCH DEVELOPMENT CORPORATION
101 Newington Causeway
London SE1 6BU(GB)

(72) Inventor: Goldsworthy, Andrew
6 Sandall Road
London W5 1JD(GB)

(72) Inventor: Rathore, Keerti Singh
36 Stanley Road
New Southgate London N11 2LE(GB)

(74) Representative: Percy, Richard Keith
Patent Department National Research Development
Corporation 101 Newington Causeway
London SE1 6BU(GB)

(54) **Method of plant tissue culture.**

(57) Plant tissue culture does not always proceed rapidly and the induction of differentiation is often difficult. It has been found that growth and/or differentiation can be stimulated, in some instances very dramatically, by application of a small electrical current to the cultures, preferably in conjunction with an appropriate auxin of a kind which undergoes polar transport, such as IAA. The invention is particularly applicable to callus culture.

METHOD OF PLANT TISSUE CULTURE

Background of the invention

1. Field of the invention

This invention relates to a method of plant tissue culture.

Broadly, plant tissue culture can be divided into three main categories - callus culture, suspension culture and the culture of organs such as meristems, roots etc. The present invention is concerned only with the culture of undifferentiated plant tissue and therefore the third category, involving culture of differentiated tissue, is not relevant.

Callus culture is a means of growing a more or less undifferentiated tissue derived from various parts of a plant on a suitable medium. It is of particular importance because genetically engineered material is normally first obtained in the form of a callus before differentiation.

Suspension culture involves culturing cells or small clumps of cells in an agitated medium. This is a convenient method of culture because it makes use of fermentors similar to those used in the culture of bacteria. However, the plant cells do not in general stand up well to rough handling. Thus it is often advantageous to protect the plant cells in some way, e.g. by growing them on a support and circulating nutrient solution over it. By way of example, UK Patent Specifications 1494534 and 1520217 (Wyo-Ben Products, Inc) describe water-swellable inorganic support materials for this purpose. Various other immobilising means including gels, beads, matrices and so on have been advocated.

The present invention is applicable to culture of initially undifferentiated plant tissue in which the tissue is immobilised, including callus culture and protected suspension culture as described above. The term "tissue" is used in a broad general sense regardless of whether individual cells in the plant material are connected together or not.

For the purposes of this specification, tissue is regarded as undifferentiated when there is no obvious formation of recognisable plant organs such as leaves, buds or roots. A mere difference between individual cells indicating that some have specialised to form particular functions is not considered to be differentiation for the purposes of this specification. For example a callus can become green in parts as some cells develop a photosynthetic speciality, but the callus is considered to be undifferentiated.

2. Description of the prior art

There have been many literature papers describing the experimental measurement of natural electrical currents in growing plants and of the experimental application of electrical potentials to growing plants. Reviews of the subject have been made by L.F Jaffe and R. Nuccitelli, Ann. Rev. Biophys. Bioeng., 6, 445-476 (1977) and by H.W. Ellis and E.R. Turner, Science Progress, Oxford, 65, 395-407 (1978). W.W. Webster and A.R. Schrank, Arch. Biochem. Biophys., 47, 107-118 (1953) have described a change in the direction of growth in barley coleoptiles by application of a transverse electrical potential, which appears to cause lateral transport of IAA in the coleoptile. J.D. Black et al., Canadian Journal of Botany, 49, 1809-1815 (1971) have shown that the rate of linear growth of tomato plants could be stimulated by various amounts in the range 5-30% by passing a small current between the plant and the soil. B.O. Bratton et al., J. Exp. Bot., 28, 338-344 (1977) showed that small currents applied to tomato plants between stem and soil altered the levels of IAA and peroxidase in different parts of the plant. J. Zvara et al., Z. Pflanzenphysiol., 96, 251-260 (1980), report uptake of ions by barley plants placed in a wholly external electrical field. L.F. Jaffe and R. Nuccitelli, loc. cit. speculate that weak electric currents flow through cells of the Fucus egg and other unicells to control their growth and differentiation.

Despite the existence of this body of literature, or because of the difficulty of drawing useful conclusions from the experimental evidence which it presents, very little study has been made on electrical effects in relation to plant tissue culture.

A.I. Litinov et al., Fiziol. Rast., 26, 1203-1214 (1979), abstracted in Biological Abstracts 70, 67439, describe measurements on tobacco plant cells in suspension culture. Intracellular concentrations of potassium ions and the potential difference between the vacuole of the cell and the external solution were measured. P. Burgess and P.J. Linstead, Planta 156, 241-248 (1982) have described experiments with individual protoplasts of Physcomitrella patens, a kind of moss. These protoplasts are capable of rapid formation of a fibrillar cell wall, followed by emergence of filamentary outgrowth from the wall. When the protoplasts were grown in an electric field, filaments emerged in the direction of the positive pole of the field. However, the applicants are not aware of any reference describing application of an electrical potential to plant tissue cells.

It will be appreciated that application of an electrical current to plant tissue for cultural purposes, in the hope of improving growth, immediately introduces complications into the procedure. Therefore, unless there is reason to believe that growth or differentiation will be improved very spectacularly it would not be an expedient worth trying. The literature papers describing studies on growing plants or on protoplasts have not suggested that application of electric currents is likely to bring about dramatic effects in plant tissue culture.

Summary of the invention

The present invention arises from the very surprising finding that application of a small electric current causes a very large stimulation of growth and/or differentiation. The best results obtained to date in the present invention indicate an increase in differentiation of about 600% and an increase in growth of about 75%. The magnitude of these effects is astonishing.

The present invention provides a method of stimulating growth and/or differentiation of plant tissue, in the culture of plant callus or of undifferentiated, immobilised plant cells, which comprises passing an electric current through it. Preferably an auxin capable of undergoing polar transport within plant tissue, especially 3-indoleacetic acid (IAA), is added to the culture.

The electrical current can be applied in any manner such that it passes through the plant material under culture. If both electrodes are placed in a callus, only weak stimulation of growth occurs, the best effects of the invention being obtained by placing one electrode within the plant tissue and the other externally, i.e. in the culture medium. It has been found that the stimulatory effects of the electrical current are greatest when the plant tissue electrode is negative.

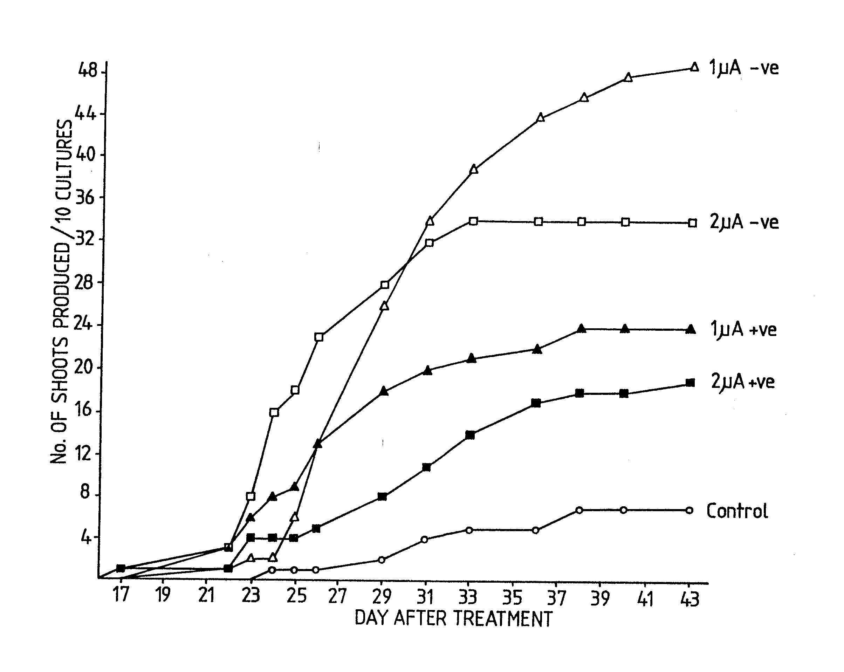

Brief description of the drawing

The accompanying drawing is a plot of a measure of differentiation along the ordinate and time (days after the beginning of electrical stimulation) on the abscissa. The drawing is referred to in Example 1.

Description of the preferred embodiments

The electrical current to be applied need only be small. In laboratory experiments conducted with small pieces of callus each initially weighing 145 mg on average, a current of 1 microamp was found to give very good results but increasing it to 2 microamps was less stimulatory to growth and differentiation. Translating these figures into current densities is difficult, since as the callus grows it receives a reduced current density and since anyway it is of an irregular shape with a range of different diameters. The shape of the current path can be approximated to a cone with the electrode at the apex and spreading outward to a circular base. The 1 microamp current corresponded initially to about 10 microamps/$cm^2$ current density at a cross-section at the base of the cone. This is an estimate made at the beginning of the experiment while the callus was small. During growth the base area expanded, but the current was not increased and so the current density at the base fell, reaching about 0.25 microamps/$cm^2$ at the end of the experiment. Since the current can be held constant, one needs to consider a time-average current density. Generally stated, an appropriate time-averaged range of current densities over the cone base is likely to be between 0.01 and 100 microamps/$cm^2$. Some effect, albeit small, is likely to be obtained at very low current densities, e.g. of 0.1 microamps/$cm^2$ or less, while the effect probably tapers away at current densities appreciably above 20 microamps/$cm^2$ (corresponding to the 2 microamp current used experimentally). Broadly stated, a preferred lower limit is 0.2 and more preferred 0.5 microamps/cm$^2$. Obviously, high currents likely to generate substantial quantities of electrolysis products, or likely to harm the plant tissue by excessive heating effects must be avoided, but this is believed inherent in the definition of the invention as a method of tissue culture. The tolerable upper limit will vary according to the kind of plant, configuration and density of the tissue mass under cultivation, position of electrodes, direction of the current and doubtless other factors. Broadly stated, a preferred upper limit is 30 and more preferred 15 microamps/cm$^2$. All these suggested current densities are time-averaged. It will be appreciated that the current can be increased as the callus grows in order to even out current density fluctuations.

Whether the invention is of more benefit in obtaining growth of the tissue or obtaining differentiation will depend on the chemical composition of the culture medium. The invention is expected to be of particular value in stimulating differentiation of those plants in which it has been difficult to obtain differentiation by conventional tissue culture, for example many cereals.

In order to stimulate growth, 3-indoleacetic acid (IAA), or another appropriate plant auxin is normally required in the culture medium. Growth stimulation was only obtained in the presence of auxin and did not occur if 2,3,5-triiodobenzoic acid (TIBA), a known inhibitor of the polar transport of auxin, was also present. Several different auxins have been tested for their ability to support the electrical growth stimulation. Of those tested, IAA was the most effective, 1-napthaleneacetic acid (NAA) less effective and 2,4-dichlorophenoxyacetic acid (2,4-D) virtually ineffective. The ability to support the effect appears to correlate with the reported ability of the various auxins to undergo polar transport in plant tissues, IAA being transported much more rapidly than 2,4-D.

Polar transport of an auxin is defined herein as the translocation of an auxin within a plant in a direction away from its apex and towards the base. It is normally unidirectional, at least in the apical regions.

The auxin will normally be added (supplied exogenously) to the culture medium, but in some plants which synthesise a large amount of auxin this might not be necessary.

IAA is a natural product and a food constituent, but the use of synthetic auxins is not to be advocated without adequate toxicological safeguards if the culture products are for food or drug use. The stimulatory effect on differentiation requires a lower ratio of IAA:kinetin than the stimulatory effect on growth, as in conventional culture. Sometimes it is desired to stimulate differentiation, but not necessarily growth. In these circumstances particularly, it should be possible to dispense with the auxin.

In callus culture by the method of the invention, the medium is solid and non-biodegradable, e.g. agar, and contains nutrient(s) and growth factor(s). Alternatively a solid support such as a plastics mesh or a filter paper can be used in conjuction with a liquid medium containing nutrient(s) and growth factor(s).

The method of the invention is not limited to any one kind of plant or the callus derived from any particular part of the plant. Nor is it confined to callus culture, but is considered generally applicable mutatis mutandis to cultures derived from cell suspensions in which the cells are immobilised.

The culture can be carried out under any of the usual conditions, e.g. at a temperature of 20-30°C, preferably 25°C. Usually conditions such as a temperature as low as 15°C or as high as 35°C may be tolerable when differentiation, rather than mere growth, is the main aim. Generally stated, illumination favours differentiation and therefore light would be shone for at least a period to obtain best advantage. Weak illumination is adequate. A 16 hour photoperiod or dim continuous light will normally be appropriate. The method is operable in the dark.

Besides the plants themselves produced by the method of the invention, the invention includes the secondary metabolites, which may be of value as pharmaceuticals, perfume oils, food flavourings etc. dependent on the nature of the plant.

The following Examples illustrate the invention.

## EXAMPLE 1

Seeds of Nicotiana tabacum var. virginica (obtained from Thompson & Morgan Ltd., Ipswich, Suffolk, England) were surface sterilized for 2 minutes in 70% ethanol and then for 30 minutes in sodium hypochlorite with 3% available chlorine. They were washed 4 times with sterile tap water and grown in the dark at 25$^o$C on moist filter paper in glass petri dishes. Pieces of hypocotyl 1 cm long were excised from 7 day old seedlings and incubated on agar media in 100 mm conical flasks at 25$^o$C under fluorescent lights giving 0.35 W.m$^{-2}$ at bench level. After 5-6 weeks, the extensive callus growth which proliferated from the cut surfaces of the explant were transferred to a fresh, sterilized medium. The medium used was Gamborg's B5 medium at pH 5.5 (Gamborg et al., Exp. Cell Res., 50, 151-158, (1968)), solidified with 1% agar and supplemented with 2% sucrose and also containing 1 ppm 2,4-dichlorophenoxyacetic acid (2,4-D) and 0.1 ppm kinetin. After a further 5 weeks, the experimental inocula, each averaging about 145 mg, were cut from the newly-proliferated outer regions of the callus.

These were transferred to a fresh medium of the same composition as the previous medium except that the 2,4-D and kinetin were replaced by 0.03 ppm 3-indoleacetic acid (IAA) and 1 ppm kinetin. The callus pieces were kept under dim light (0.35 W.m$^{-2}$) for a week before the electrodes were implanted.

The current was passed through the callus from two stainless steel electrodes, one in the callus, and the other in the external medium about 2 cm away. Both were formed by inserting the bared ends of 15 cm lengths of PTFE-insulated stainless steel wire (bared diameter 0.25 mm, coated diameter 0.33 mm) to a depth of 2-3 mm. The wires were bent over the neck of the flask (which was then covered with aluminium foil) and connected to the external circuit. The current was applied continuously from a d.c. power supply via a separate 10 megohm resistor to each flask. Because the value of this resistor was high compared to the variable resistance of the tissue, a steady current was maintained which remained constant to within 2% throughout the experiment. The current was adjusted by regulating the voltage of the power supply and monitored at weekly intervals by an Ohm's law calculation from the voltage drop across the 10 megohm resistor recorded by a digital voltmeter. Two current levels were tested, 1 and 2 microamps (measured as 10 and 20 volts respectively across the 10 megohm resistor) at each polarity. Each treatment contained 10 replicate flasks with a further 10 flasks serving as the control in which the electrodes were implanted but left unconnected.

During the application of the electric current, the cultures were grown in a constant temperature room at $23^{\circ}$C under fluorescent illumination at 7.2 W.m$^{-2}$ with a 16 hour photoperiod. The cultures were periodically checked for differentiation and the total number of shoots per treatment recorded. Photographs were taken on the 26th day of treatment to show the position of the regenerated shoots in relation to the electrical polarity.

The results are shown in the accompanying drawing in which the number of shoots per 10 cultures is plotted against days after treatment. Obvious differentiation did not begin for about 3 weeks, but after this period, shoot formation in all the electrical treatments was much more rapid than in the controls. The treatments in which the callus was made negative gave the best results with a 7-fold stimulation at 1 microamp and a 5-fold stimulation at 2 microamps. Stimulations of the order of 3-fold were obtained when the callus was made positive. At either polarity, the higher current was less effective.

A very interesting observation is that in the callus negative treatments, the newly formed shoots were concentrated around the electrode but in the callus positive treatments they were concentrated at the base of the callus next to the medium, i.e. in both cases they were in the most negative region of the callus.

### EXAMPLE 2

Example 1 was repeated except that for electrical stimulation the concentrations of IAA and kinetin were changed to 4 ppm IAA and 0.3 ppm kinetin. This change favours growth of the tissue over shoot formation.

The effect of passing current between the medium and the callus on callus growth is shown in Table 1 below.

The weights of tissue and their standard errors after 22 days, expressed as a mean for at least 9 replicates, are given. The sign indicates the polarity of the electrode inserted in the callus. Similar electrodes were inserted into the controls but were left unconnected.

TABLE 1

|  | Grams fresh weight | Grams dry weight |
|---|---|---|
| Control | 2.69 ± 0.46 | 0.091 ± 0.011 |
| +ve 1 microamp | 3.22 ± 0.48 | 0.101 ± 0.014 |
| +ve 2 microamps | 1.85 ± 0.25 | 0.076 ± 0.006 |
| -ve 1 microamp | 4.74 ± 0.29 | 0.161 ± 0.008 |
| -ve 2 microamps | 3.88 ± 0.47 | 0.143 ± 0.015 |

EXAMPLE 3

Example 1 was repeated except that during electrical stimulation the cultures were grown in the dark. Results are shown in Tables 2 and 3 below.

TABLE 2

Time course of shoot formation from 12 tobacco callus cultures in dark. The figures are total number of shoots.

| Day | 32 | 40 | 55 | 84 |
|---|---|---|---|---|
| Control | 0 | 1 | 4 | 37 |
| +ve 1μA | 0 | 0 | 18 | 146 |
| -ve 1μA | 7 | 25 | 32 | 151 |
| +ve 2μA | 0 | 0 | 11 | 84 |
| -ve 2μA | 0 | 0 | * | 94 |

*The number of shoots produced was so large that it was impossible to count them without stopping the experiment.

## TABLE 3

Percentage of cultures showing shoot formation in tobacco callus cultures in dark.

| | Day 32 | 40 | 55 | 84 |
|---|---|---|---|---|
| Control | 0 | 1 | 4 | 37 |
| +ve 1µA | 0 | 0 | 58 | 83 |
| -ve 1µA | 25 | 25 | 41 | 58 |
| +ve 2µA | 0 | 0 | 33 | 75 |
| -ve 2µA | 0 | 0 | 41 | 50 |

## EXAMPLE 4

Wheat (Triticum aestivum var. Maris Huntsman) seeds were surface serilised for 1 minute in 70% ethanol and then for 15 minutes in sodium hypochlorite with 7% available chlorine. They were washed 4 times with sterile distilled water and left to imbibe overnight in the refrigerator. Next day the embryos were excised from the seeds and placed in petri dishes on B5 medium containing 1% agar, 2% sucrose, 50 ppm glutamine, 10 ppm ascorbic acid and 1 ppm 2,4-D at pH 5.7. After 6 weeks the resulting callus was transferred to flasks contianing 25 ml of medium of the same composition except that 2,4-D was replaced by 1 ppm IAA and 1 ppm zeatin. After 4 days electrodes were inserted and the experiment was conducted as described in Example 1. The results of the effect of current on shoot and root formation are shown in Tables 4, 5 and 6 below.

## TABLE 4

Number of roots per culture and standard errors after 42 days of treatment.

| Control | $7.36 \pm 1.35$ | |
|---|---|---|
| +ve 1µA | $14.16 \pm 2.32$ | $P = 0.02$ |
| -ve 1µA | $4.45 \pm 1.11$ | |
| +ve 2µA | $15.25 \pm 2.76$ | $P = 0.02$ |
| -ve 2µA | $6.0 \pm 1.40$ | |

TABLE 5

Estimated total root length (mm) per culture after 42 days of treatment.

| | |
|---|---|
| Control | 73.5 ± 16.95 |
| +ve 1µA | 116.5 ± 32.10 |
| -ve 1µA | 28.5 ± 10.90    P = 0.05 |
| +ve 2µA | 87.5 ± 17.50 |
| -ve 2µA | 59.0 ± 33.38 |

TABLE 6

Number of shoots from 12 cultures after 57 days of the treatment.

| | |
|---|---|
| Control | 0 |
| +ve 1µA | 2 |
| -ve 1µA | 0 |
| +ve 2µA | 2 |
| -ve 2µA | 0 |

EXAMPLE 5

The experiment described in Example 4 was repeated with wheat (Triticum aestivum var. Sicco) except that the callus was grown on 2,4-D medium for about 100 days instead of 6 weeks. The longer period of growth on the 2,4-D was to reduce the morphogenetic potential of the callus for shoot formation. However, during this period the cultures formed roots. These were removed before the transfer to the IAA/zeatin medium. Results on the effect of current on shoot formation are shown in Table 7 below.

- 12 -

### TABLE 7

Number of shoots from 12 cultures after 41 days of the treatment.

| Control | 0 |
|---|---|
| +ve 1μA | 0 |
| -ve 1μA | 0 |
| +ve 2μA | 7 |
| -ve 2μA | 6 + 2S |

S = Green spots on the callus which normally develop into shoots at a later stage.

70B

- 13 -

CLAIMS

1.    A method of stimulating growth and/or differentiation of plant tissue, in the culture of plant callus or of undifferentiated, immobilised plant cells, characterised in that an electric current is passed through the culture.

2.    A method according to Claim 1, characterised in that an auxin capable of undergoing polar transport is supplied exogenously to the culture.

3.    A method according to Claim 2, characterised in that the auxin comprises 3-indoleacetic acid.

4.    A method according to Claim 1, 2 or 3 characterised in that the electric current is passed at a current density averaged over the time of passage and measured over the largest cross-sectional area of the tissue of from 0.01 to 100 microamps/cm$^2$.

5.    A method according to Claim 4 characterised in that the current density is from 0.2 to 15 microamps/cm$^2$.

6.    A method according to any preceding claim characterised in that the current is passed between a first electrode external to the plant tissue and a second electrode within it.

7.    A method according to Claim 6 characterised in that the second electrode is negatively charged with respect to the first.

8.    A method according to any preceding claim characterised in that it is carried out under weak illumination or in the dark and at a temperature of from 15 to 35$^\circ$C.

9.    A method according to any preceding claim characterised in that it is carried out at a temperature of from 20 to 30$^\circ$C.

70B

1/1

0142987